# EUROPEAN PATENT SPECIFICATION

(11) **EP 1 678 301 B1**
(45) Date of publication and mention of the grant of the patent: **09.12.2009**
(21) Application number: 04761250.2
(22) Date of filing: 08.09.2004
(51) Int. Cl.: C12N 15/11, C12N 15/31, C12N 15/70, C12N 15/74, A61K 39/08

(54) **INDUCIBLE BACTERIAL EXPRESSION SYSTEM UTILISING SSPA PROMOTER FROM SALMONELLA**
INDUZIERBARES BAKTERIELLES EXPRESSIONSSYSTEM MIT NUTZUNG DES SSPA-PROMOTORS AUS SALMONELLA
SYSTEME D'EXPRESSION BACTERIEN INDUCTIBLE UTILISANT UN PROMOTEUR SSPA DE LA SALMONELLE

(30) Priority: 08.09.2003 AU 2003904873
(43) Date of publication of application: 12.07.2006
(73) Proprietor: THE UNIVERSITY OF QUEENSLAND, Brisbane, Queensland 4067 (AU)
(72) Inventor: TERRY, Tasmin, Deborah, St Lucia, Queensland 4067 (AU); JENNINGS, Michael, Paul, Carina, Queensland 4152 (AU)
(74) Representative: ABG Patentes, S.L.
(86) International application number: PCT/AU2004/001213
(87) International publication number: WO 2005/024019

(56) References cited:
- WO-A-00/52181
- WO-A-01/00791
- WO-A-02/20789
- WO-A-02/38739
- JENNINGS M P ET AL: "Regulation of the ansB gene of Salmonella enterica" MOLECULAR MICROBIOLOGY, BLACKWELL SCIENTIFIC, OXFORD, GB, vol. 9, no. 1, July 1993 (1993-07), pages 165-172, XP002977901 ISSN: 0950-382X
- GAZIT G ET AL: "USE OF THE GLUCOSE STARVATION-INDUCIBLE GLUCOSE-REGULATED PROTEIN 78 PROMOTER IN SUICIDE GENE THERAPY OF MURINE FIBROSARCOMA" CANCER RESEARCH, AMERICAN ASSOCIATION FOR CANCER RESEARCH, BALTIMORE, MD, US, vol. 59, July 1999 (1999-07), pages 3100-3106, XP002931969 ISSN: 0008-5472
- HANSEN ANNE-MARIE ET AL: "Escherichia coli SspA is a transcription activator for bacteriophage P1 late genes." MOLECULAR MICROBIOLOGY JUN 2003, vol. 48, no. 6, June 2003 (2003-06), pages 1621-1631, XP002465559 ISSN: 0950-382X
- TERRY TAMSIN D ET AL: "Investigation of ansB and sspA derived promoters for multi- and single-copy antigen expression in attenuated Salmonella enterica var. typhimurium." VACCINE 22 AUG 2005, vol. 23, no. 36, 22 August 2005 (2005-08-22), pages 4521-4531, XP002465560 ISSN: 0264-410X
- DATABASE GENBANK [Online] 13 January 1999 DILLINGER, D. ET AL.: 'Salmonella Pullorum stringent starvation protein (SSP) gene', XP008092881 Database accession no. (AF105252) & DILLINGER, D. ET AL.: 'Salmonella pullorum stringent starvation protein (SSP) gene' 13 January 1999,
- WILLIAMS, M.D. ET AL.: 'Starvation-induced expression of SspA and SspB: the effects of a null mutation in sspA on Escherichia coli protein synthesis and survival during growth and prolonged starvation' MOLECULAR MICROBIOLOGY vol. 11, no. 6, 1994, pages 1029 - 1043, XP008067544
- DATABASE GENBANK [Online] 12 September 1993 SERIZAWA, H.ET AL.: 'E.coli SSP gene for stringent starvation protein', XP008092882 Database accession no. (X05088) & SERIZAWA, H. ET AL.: 'E.coli SSP gene for stringent starvation protein' 12 September 1993,
- WILLIAMS, M.D. ET AL.: 'The possible use of the stringent starvation protein ssp promoter of Esscherichia coli in an expression system for cloned genes' ABSTRACTS OF PAPERS AMERICAN CHEMICAL SOCIETY vol. 199, no. 1-2, 1990, page 124, XP008092288
- SERIZAWA, H. ET AL.: 'Structure of the gene for the stringent starvation protein of Esscherichia coli' NUCLEIC ACIDS RESEARCH vol. 15, no. 3, 1987, pages 1153 - 1163, XP001208060

## Description

### FIELD OF THE INVENTION

THIS INVENTION relates to an inducible bacterial promoter and expression system suitable for expression of heterologous nucleic acids and proteins, without being limited thereto. In particular, this invention relates to the use of an expression construct comprising a bacterial stringent starvation protein promoter for use in a pharmaceutical composition. The pharmaceutical composition is suitable for use as an immunotherapeutic composition and in particular, a vaccine delivery system. The expression construct is capable of being maintained extra-chromosomally or by integration into a bacterial host genome, whereby proteins may be expressed.

### BACKGROUND OF THE INVENTION

For some time, bacterial systems have found widespread application in general protein expression as well as in the treatment of various protein-related conditions. Successful applications of such pharmaceutical compositions in the form of bacterial systems are used for vaccine delivery in immunization regimes. Attenuated bacteria such as *Salmonella* have been used for this purpose, both for providing immunization against the attenuated bacteria itself, and for delivery of heterologous proteins useful as immunogens.

In order to deliver heterologous proteins, bacterial expression vectors have been devised in the form of extra-chromosomal plasmid vectors and as vectors which are integrated into bacterial chromosomal DNA (Strugnell et al., 1990, Gene 88 57).

Multi-copy, extra-chromosomal plasmid vectors with constitutive promoters tend to provide higher levels of expression, particularly during bacterial propagation, but can be inherently unstable and may be lost during propagation.

Vectors or antigens expression cassettes integrated as a single copy in the bacterial chromosome have greater stability, but tend to achieve much lower levels of expression than do extra-chromosomal vectors.

Accordingly, regulatable promoters have been sought which display minimal activity during bacterial growth but increased activity in host tissues (Chatfield et al., 1992, Biotechnology 10 888). Generally, anaerobically-regulated promoters are relatively inactive during bacterial growth where aerobic conditions prevail.

Examples of bacterial regulatory promoters are the ansB promoter (International Publication WO 02/38739) and the *E*. *coli* sspA promoter (Williams et al., 1994, Molecular Microbiology 11 1029; Williams et al., 1990, Abstracts of American Chemical Society 199 124).

The *sspA* gene encodes the stringent starvation protein SSP in *E. coli* (Serizawa & Fukuda, 1987, Nucl. Acids Res. 15 1153) and *Salmonells enterica* (Genbank Accession AF105252). The *E. coli* regulatory *sspA* promoter is induced under conditions of extreme starvation, where up to 50% of total protein synthesis in the cell is derived from this promoter. Under extreme starvation, the relative synthetic rate of SSP increases 15-20 fold in a stringent strain, making up at least 50% of total protein synthesis (Reeh *et al.,* 1976 Molecular and General Genetics **149** 279) and renders this protein synthesis dramatically stimulated by starvation.

Although the function of SSP is unknown, it is suggested it may be involved as a mediating factor between ppGpp and RNA polymerase. The protein SSP is not required for normal growth, however in long term (10 day) amino acid starvation, *ΔsspA* mutants do not survive as long as *sspA⁺* cultures (Williams *et al.,* 1994, *supra*). SSP protein expression is dependent on a number of factors including growth rate, the presence of a functional *relA* gene and is induced by glucose, nitrogen, phosphate or amino acid starvation. However, *ΔsspA* mutants change the expression of at least 11 other proteins (Williams *et al.,* 1994, *supra*).

The *E.coli* sspA promoter (PsspA) has been isolated and genetically mapped (Fakuda et al., Mol Gen Genet., 1985, 201:151-157; Williams *et al.,* 1994, *supra*). In studies by Williams *et al.* 1994, *supra,* starvation-induced expression from a single copy plasmid with the promoter P*sspA* fused to a promoterless *lacZ* gene was demonstrated, however unlike other stationary-phase-inducible promoters, starvation-induced expression of the SspA protein was not observed in any multi-copy plasmid-encoded system.

### SUMMARY OF THE INVENTION

The present invention relates to a new stringent starvation protein *Salmonella sspA* promoter and expression system suitable for expression of heterologous nucleic acids and proteins. While applicable to general protein expression, this invention also relates to the use of an expression vector or construct comprising either a *Salmonella* or an *E*. *coli sspA* promoter and their use in a pharmaceutical composition, which promoters demonstrate surprising and hitherto unexpected efficacy for heterologous protein expression. The pharmaceutical composition is suitable for use as an immunotherapeutic composition and in particular, as a vaccine delivery system. The expression construct is capable of being maintained extra-chromosomally or by integration into a bacterial host genome, whereby proteins may be expressed.

In a first aspect of the present invention resides in an isolated nucleic acid comprising at least one promoter having a nucleotide sequence set forth in SEQ ID NO: 1.

In a second aspect, there is provided an isolated nucleic acid comprising at least one promoter active fragment of the nucleotide sequence set forth in SEQ ID NO: 1, wherein said promoter active fragment has at least 50% of the activity of a regulatable promoter comprising the nucleotide sequence of SEQ ID NO:1.

In a third aspect, the present invention provides an isolated nucleic acid comprising a promoter, or promoter active fragment thereof, having at least 60 % nucleotide sequence identity with the nucleotide sequence set forth in SEQ ID NO: 1, but excluding the nucleotide sequence set forth in SEQ ID NO:2, wherein said promoter active fragment has at least 50% of the activity of a regulatable promoter comprising the nucleotide sequence of SEQ ID NO:1.

In a fourth aspect, the present invention provides a chimeric gene comprising a promoter including a nucleotide sequence set forth in SEQ ID NO: 1, or a promoter active fragment or homolog thereof, and a heterologous nucleic acid.

In a fifth aspect, the invention provides an expression vector comprising an isolated nucleic acid according to the first, second or third aspects.

Preferably, the promoter is a regulatory component of a *Salmonella sspA* gene and the bacteria is of a *Salmonella* strain.

In one embodiment, the expression vector is chromosomally integrated and maintainable in a bacterium.
In another embodiment, the expression vector is maintainable extra-chromosomally in a bacterium.

In a sixth aspect, the invention provides an expression construct comprising an expression vector according to the fifth aspect and a heterologous nucleic acid operably linked to the promoter.

In a seventh aspect, the invention provides a bacterium transformed with the expression construct of the sixth aspect.

Preferably, the bacterium is of a strain of *Salmonella* sp.

In one embodiment, the expression construct is chromosomally-integrated and maintained in the bacterium.

In another embodiment, the expression construct is maintained extra-chromosomally in the bacterium.

In an eighth aspect, the present invention provides an immunotherapeutic composition comprising an expression construct that comprises a promoter of bacterial origin which is regulatable by starvation conditions and comprises: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2; which is operably linked to a heterologous nucleic acid encoding an immunogenic protein.

Preferably the immunotherapeutic composition is a vaccine.

In a ninth aspect, the present invention provides an immunotherapeutic composition comprising a bacterium transformed with an expression construct having a promoter of bacterial origin which is regulatable by starvation conditions and comprises: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2; which is operably linked to a heterologous nucleic acid encoding an immunogenic protein together with a pharmaceutically-acceptable carrier, diluent or excipient.

In one particular embodiment, the immunotherapeutic composition is a vaccine.

Preferably, the bacterium is attenuated.

In particular embodiments, the bacterium is of a strain of *Salmonella* sp.

In one embodiment, the expression construct is chromosomally-integrated and maintained in the bacterium.

In another embodiment, the expression construct is maintained extra-chromosomally in the bacterium.

In a tenth aspect, the present invention provides the use of an expression construct having a promoter of bacterial origin regulatable by starvation conditions and comprising: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2; operably linked to a heterologous nucleic acid that encodes an immunogenic protein suitable for therapeutic and/or prophylaxis of one or more disease conditions responsive to immunotherapy in a host, for the preparation of a medicament for the treatment and/or prophylaxis of one or more disease conditions.

Preferably, the host is a human.

In an eleventh aspect, the present invention provides the use of an expression construct having a promoter of bacterial origin regulatable by starvation conditions and comprising: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2 operably linked to a heterologous nucleic acid that encodes an immunogenic protein, for the preparation of a medicament for vaccinating a host.

The expression construct is preferably present in an attenuated bacterium.

Preferably, the host is a human.

### BRIEF DESCRIPTION OF THE TABLES AND FIGURES

**TABLE 1:** Sequences of primers used in the construction of expression systems (plasmids and chromosomally integrated constructs).
**TABLE 2:** Bacterial strains and plasmids used in testing the expression system.

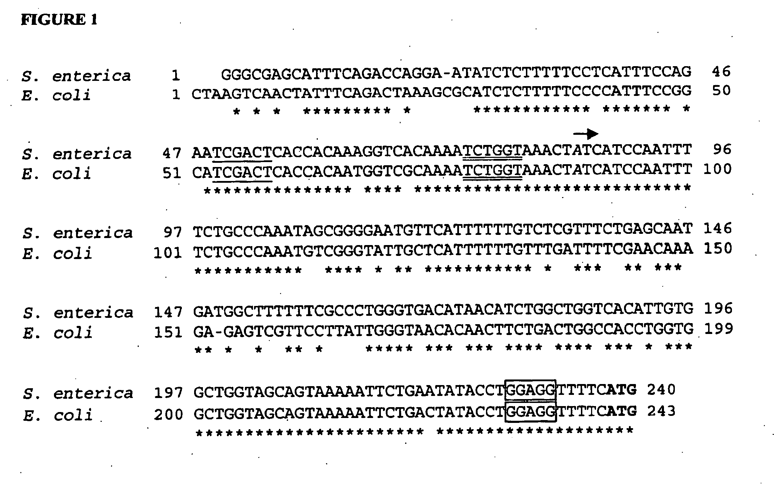

**FIG. 1****:** Alignment of the respective nucleotide sequences of the *sspA* promoters from *S. enterica* (SEQ ID NO: 1) and *E. coli* (SEQ ID NO: 2). The -35 (single underline), -10 (double underlined), transcriptional start (letter A in italics + arrow), ribosomal binding site (boxed) and start codon (ATG in bold) are marked. Nucleotides that are identical in the two promoters are marked "*". The *E. coli sspΛ* promoter and gene sequence may be found in Genbank under Acession number X05088. Note that the sequences in the vectors differ by one base pair from the sequences shown in FIG. 1 as the primer used to amplify the sequences introduces an *Nco*I site at the 3' end of both promoters resulting in the sequence TTTCCATG rather than TTTTCATG in the wild-type.

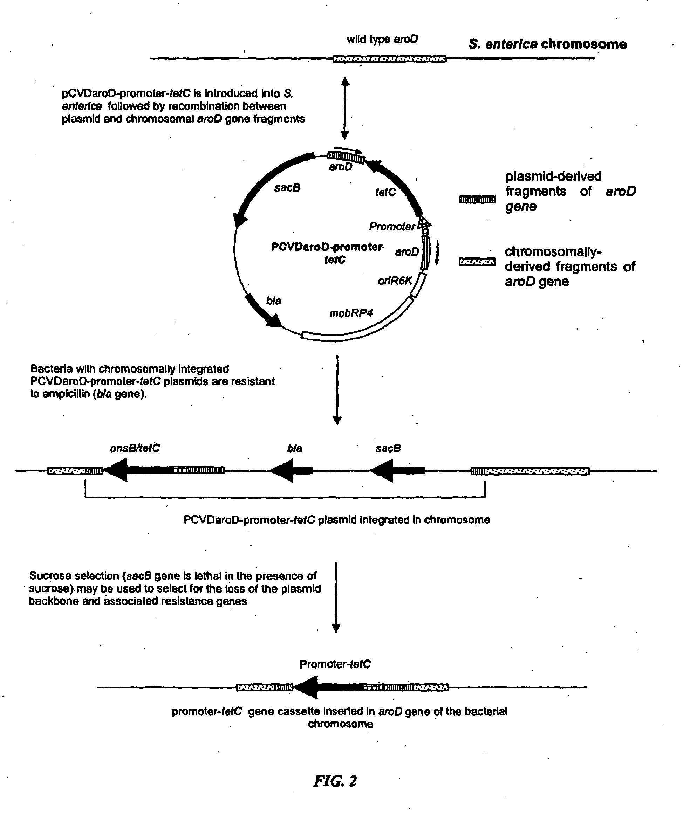

**FIG. 2****:** Schematic summary of construction of a *S*. *enterica* strain having a chromosomally-integrated promoter-*tetC* cassette.

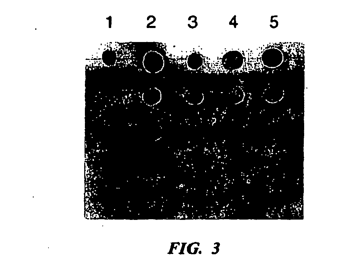

**FIG. 3****:** Comparison of expression of fragment C driven by chromosomally-integrated or plasmid encoded *S*. *enterica sspA* promoter in *S*. *enterica* serovar *typhimurium* strain STM1/Rif. Expression constructs consisted of the fragment C gene downstream of the *S. enterica sspA* promoter as a plasmid (pKKsspAtetC, Strain JD06), as a plasmid integrated into the *aroD* gene of the chromosome (pCVDaroDsspAtetC, Strain JE01), or a double-crossover (only the *sspA-tetC* cassette in the chromosome, JD09). Cultures were grown to an OD at 600 nm of 0.5 in 20 ml of LB (supplemented with 100 µg/ml Ampicillin where appropriate) in a 50 ml Falcon Tube. Cells were pelleted by centrifugation, resuspended in PBS, sonicated and the lysatc clarified. Serial four-fold dilutions were dotted onto a nitrocellulose filter. The filter was blocked overnight with PBS/5% skim milk, incubated for 1 h with 1: 2500 dilution of monoclonal anti-tetanus toxin fragment C antibody (Roche) and washed 3 times with PBS. The filter was incubated with sheep anti-mouse IgG alkaline phosphatase antibody for 1 h (1: 5000 dilution in PBS/5% skim milk). After incubation the blot was washed 3 times with PBS, rinsed with alkaline phosphatase buffer and developed using nitro blue tetrazolium/5-brom-4-chloro-3-indolyl phosphate solution in alkaline phosphatase buffer (100 mM NaCl, 5 mM MgCl₂, 100 mM Tris.Hcl (pH 9.5). Dot blotting was used as the monoclonal anti-tetanus toxin fragment C antibody is extremely sensitive to conformational changes, and so works sub-optimally in western blots where proteins have been denatured. Lane 1 - strain JA08, lane 2 - strain JD06, lane 3 - strain JE01, lane 4 - strain JD09, lane 5 - strain JB07.

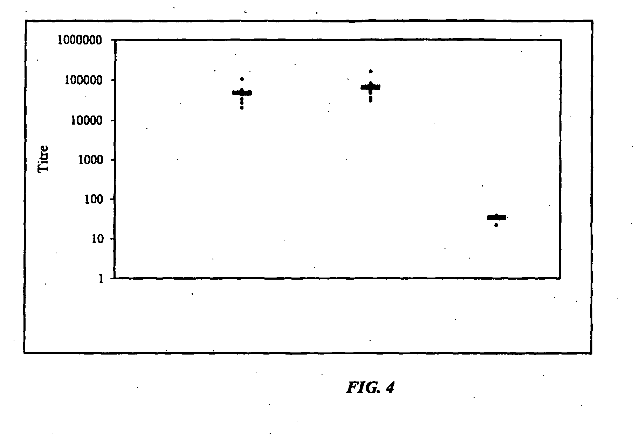

**FIG. 4****:** Immune responses raised against tetanus toxoid after oral inoculation of BALB/c mice with *Salmonella* strains containing plasmids expressing fragment C. The results for strains JB07, JB06 and JB08 are shown left to right. Two-fold serial dilutions of sera obtained from each mouse at day 49 were analysed in duplicate from a starting dilution of 1:500 (strains JB07 and JD06) or 1:20 (strain JA08). Titres were determined as the reciprocal of the dilution giving a response equal to the average of the negative control group at a dilution of 1:40 (JA08) + 2 standard deviations. The average titre for each group is shown as a black bar.

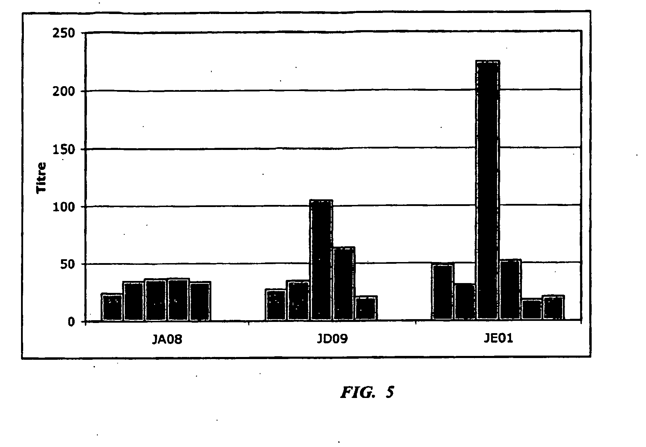

**FIG. 5****:** Immune responses raised against tetanus toxoid after oral inoculation of BALB/c mice with *Salmonella* strains expressing fragment C under the control of the *sspA* promoter located in the *Salmonella* chromosome. Two fold serial dilutions of sera obtained from each mouse at day 49 were analysed in duplicate from a starting dilution of 1: 20. Titres were determined as the reciprocal of the dilution giving a response equal to the average of the negative control group (JA08) at a dilution of 1:40 + 2 standard deviations. Titres for each individual mouse in each group are shown.

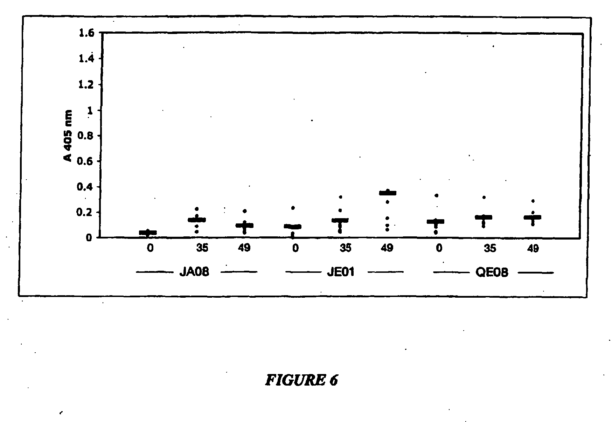

**FIG. 6****:** Comparison of immune responses raised against tetanus toxoid after oral inoculation of *Salmonella* strains expressing fragment C under the control of the *sspA* or *pagC* promoters located in the *Salmonella* chromosome into BALB/c mice. Serum samples from each mouse at day 0, 35, and 49 were analysed in duplicate at a dilution of 1:40. The average titre of each group is shown as a black bar.

### DETAILED DESCRIPTION OF THE PREFERRED EMBODIMENTS

The present invention is predicated, at least in part, by the present inventors' discovery of the unexpected utility of an *ssp*A promoter in an attenuated bacterial vaccine delivery system. Further, the inventors have isolated a new, regulatable promoter obtained from *Salmonella* having sequence homology to the *E*. *coli sspA* promoter and which is particularly efficacious for heterologous protein expression.

The *sspA* promoter is considered to be responsive to starvation conditions, i.e. where a required amino acid is limiting, bacterial cells are able to curtail RNA accumulation. This is characterised by the production of guanosine 3'-diphosphate-5'-diphosphate which is derived from guanosine 3'-diphosphate-5'-triphosphate which is catalysed by the *relA* gene product, on the ribosome in an idling reaction of protein elongation.

For the purposes of this invention, by "*isolated*" is meant material that has been removed from its natural state or otherwise been subjected to human manipulation. Isolated material may be substantially or essentially free from components that normally accompany it in its natural state, or may be manipulated so as to be in an artificial state together with components that normally accompany it in its natural state.

The term "*nucleic acid*" as used herein designates single-or double-stranded mRNA, RNA, cRNA and DNA, said DNA inclusive of cDNA and genomic DNA.

A "*polynucleotide*" is a nucleic acid having eighty (80) or more contiguous nucleotides, while an "*oligonucleotide*" has less than eighty (80) contiguous nucleotides.

A "*probe*" may be a single or double-stranded oligonucleotide or polynucleotide, suitably labelled for the purpose of detecting complementary sequences in Northern or Southern blotting, for example.

A "*primer*" is usually a single-stranded oligonucleotide, preferably having 15-50 contiguous nucleotides, which is capable of annealing to a complementary nucleic acid "template" and being extended in a template-dependent fashion by the action of a DNA polymerase such as *Taq* polymerase, RNA-dependent DNA polymerase or Sequenase™.

*A* "*protein*" is an amino acid polymer comprising natural and/or non-natural amino acids, inclusive ofD- and *L*-amino acid configurations.

By "*polypeptide*" is also meant a "*protein*" having more than fifty (50) contiguous amino acids.

*A* "*peptide*" is a protein having up to fifty (50) contiguous amino acids.

Proteins, polypeptides and peptides may comprise natural and/or non-natural amino acids as are well known in the art.

### Nucleic Acids and promoters

The present invention provides promoters and expression vectors, and constructs comprising same, regulatable by environmental and/or other stimuli such as conditions of extreme starvation, although without limitation thereto.

SSP expression is dependent on growth rate, presence of a functional relA gene and is inducible by glucose, nitrogen, phosphate and/or amino acid starvation.

As used herein, the *E*. *coli sspA* promoter is set forth in SEQ ID NO: 2.

As used herein, "*Salmonella sspA promoter*" is used to represent the *Salmonella* promoter sequence set forth in SEQ ID NO: 1, inclusive of homologues, fragments and variants of SEQ ID NO: 1. Although not wishing to be bound by any particular theory, based on homology with the *E*. *coli sspA* promote sequence of SEQ ID NO: 2, it is proposed that the *Salmonella* promoter sequence is indeed a *Salmonella sspA* promoter. It is therefore proposed that the *Salmonella* promoter set forth in SEQ ID NO:1 may be inducible in response to extreme starvation conditions and/or other stimuli or conditions such as those mentioned above that modulate SSP expression.

It will therefore be appreciated that the invention is not predicated on the basis that the promoter sequence set forth in SEQ ID NO: 1 is necessarily starvation inducible, as it is clear that this is a hitherto undescribed promoter that has demonstrated efficacy in the Examples herein and may be useful in gene expression systems and, in particular, vaccine delivery systems.

Also contemplated are promoter-active fragments, variants and homologs of *sspA* promoters useful in vaccines and expression vectors of the invention. These include starvation-induced promoters such as *sspA* promoters from bacterial strains and species other than *E*. *coli* and *Salmonella,* and promoters which regulate expression of genes other than *Salmonella sspA.*

By *"promoter-active fragment"* is meant a fragment, portion or segment of an *sspA* promoter which has at least 1%, preferably at least 5 %, more preferably at least 25 % even more preferably at least 50 % advantageously at least 75 %, 80 %, 85 %, 90 %, 95 %, 96 %, 97 %, 98 % or at least 99 % of the activity of an *sspA* promoter. For example, referring to FIG. 1, a promoter-active fragment may essentially consist a sequence of nucleotides beginning from the transcriptional start site (denoted by the arrow) to the ATG translation start site.

The term "*variant*" encompasses nucleic acids in which one or more nucleotides have been added or deleted, or replaced with different nucleotides or modified bases (*e.g.* inosine, methylcytosine). In this regard, it is well understood in the art that certain alterations inclusive of mutations, additions, deletions and substitutions can be made to a reference nucleic acid whereby the altered nucleic acid retains a particular biological function or activity, or perhaps displays an altered but nevertheless useful activity. The term "*variant* " also include naturally occurring allelic variants.

For example, a promoter set forth in FIG. 1 (SEQ ID NO:1 or 2) may be mutated using random mutagenesis, oligonucleotide-mediated (or site-directed) mutagenesis, PCR mutagenesis and cassette mutagenesis. Oligonucleotide-mediated mutagenesis is well known in the art as, for example, described by Adelman et al., 1983, DNA 2:183 using vectors that are either derived from bacteriophage M13, or that contain a single-stranded phage origin of replication as described by Viera et al., 1987, Methods Enzymol. 153 3. Production of single-stranded template is described, for example, in Sections 4.21-4.41 of Sambrook *et al*. (1989, *supra*)*.* Alternatively, the single-stranded template may be generated by denaturing double-stranded plasmid (or other DNA) using standard techniques.

Alternatively, linker-scanning mutagenesis of DNA may be used to introduce clusters of point mutations throughout a sequence of interest that has been cloned into a plasmid vector. For example, reference may be made to Ausubel *et al.,* (John Wiley & Sons Inc NY, 1995-1999) (in particular, Chapter 8.4).

Region-specific mutagenesis and directed mutagenesis using PCR may also be employed to construct promoter variants according to the invention. In this regard, reference may be made, for example, to Ausubel *et al*., *supra,* in particular Chapters 8.2A and 8.5.

With regard to random mutagenesis, methods include incorporation of dNTP analogs (Zaccolo et al., 1996, J. Mol. Biol. 255 589) and PCR-based random mutagenesis such as described in Stemmer, 1994, Proc. Natl. Acad. Sci. USA 91 10747 and Shafikhani et al., 1997, Biotechniques 23 304. It is also noted that PCR-based random mutagenesis kits are commercially available such as the Diversify™ kit (Clontech).

Promoter-active fragments, variants and homologs of *sspA* promoters will, ordinarily, display a certain level of sequence identity with a respective nucleotide sequence set forth in SEQ ID NO: 1 or SEQ ID NO: 2, for example.

In one embodiment, homologs are isolated nucleic acids having at least 60 %, preferably at least 70 %, more preferably at least 80 %, even more preferably at least 90 %, 91 %, 92 %, 93 %, 94 %, 95 %, 96 %, 97 %, 98 % or at least 99 % sequence identity with a respective promoter sequence set forth in SEQ ID NO: 1 or SEQ ID NO: 2.

In another embodiment, homologues of the isolated nucleic acids have at least 60 % sequence identity with a respective promoter sequence set forth in SEQ ID NO: 1, but excluding the respective promoter sequence set forth in SEQ ID NO: 2.

Preferably, said variants and/or homologs are regulatable by environmental and or other stimuli that modulate SSP expression, including growth rate, presence of a functional relA gene, glucose, nitrogen, phosphate and/or amino acid starvation.

More preferably, said variants and homologs are regulatable by conditions of extreme starvation.

Terms used herein to describe sequence relationships between respective nucleic acids include "comparison window", "sequence identity", "percentage of sequence identity" and "substantial identity". Because respective nucleic acids may each comprise (1) only one or more portions of a complete nucleic acid sequence that are shared by the nucleic acids, and (2) one or more portions which are divergent between the nucleic acids, sequence comparisons are typically performed by comparing sequences over a "comparison window" to identify and compare local regions of sequence similarity. A *"comparison window"* refers to a conceptual segment of typically at least 6, 10, 12 or 20 contiguous residues that is compared to a reference sequence, such as used in FASTA comparisons. The comparison window may comprise additions or deletions (*i.e*., gaps) of about 20 % or less as compared to the reference sequence (which does not comprise additions or deletions) for optimal alignment of the respective sequences. Optimal alignment of sequences for aligning a comparison window may be conducted by computerised implementations of algorithms (Geneworks program by Intclligenetics; GAP, BESTFIT, FASTA, and TFASTA in the Wisconsin Genetics Software Package Release 7.0, Genetics Computer Group, 575 Science Drive Madison, WI, USA) or by inspection and the best alignment (*i.e*., resulting in the highest percentage homology over the comparison window) generated by any of the various methods selected. Reference also may be made to the BLAST family of programs as for example disclosed by Altschul et al., 1997, Nucl. Acids Res. 25 3389.

A detailed discussion of sequence analysis can be found in Unit 19.3 of CURRENT PROTOCOLS IN MOLECULAR BIOLOGY Eds. Ausubel *et al. supra.*

The term "*sequence identity*" is used herein in its broadest sense to include the number of exact nucleotide matches having regard to an appropriate alignment using a standard algorithm, having regard to the extent that sequences are identical over a window of comparison. Thus, a "*percentage of sequence identity*" is calculated by comparing two optimally aligned sequences over the window of comparison, determining the number of positions at which the identical nucleic acid base (*e.g.*, A, T, C, G, I) occurs in both sequences to yield the number of matched positions, dividing the number of matched positions by the total number of positions in the window of comparison (*i.e*., the window size), and multiplying the result by 100 to yield the percentage of sequence identity. For example, "*sequence identity*" may be understood to mean the "match percentage" calculated by the DNASIS computer program (Version 2.5 for windows; available from Hitachi Software engineering Co., Ltd., South San Francisco, California, USA).

In another embodiment, homologs are isolated nucleic acids which hybridise to a respective promoter sequence as set forth in SEQ ID NO: 1 or SEQ ID NO: 2, under at least low stringency conditions, preferably under at least medium stringency conditions and more preferably under high stringency conditions.

"*Hybridise and hybridisation*" is used herein to denote the pairing of at least partly complementary nucleotide sequences to produce a DNA-DNA, RNA-RNA or DNA-RNA hybrid. Hybrid sequences comprising complementary nucleotide sequences occur through base-pairing between complementary purine and pyrimidine bases, or between modified purines (for example, inosine, methylinosine and methyladenosine) and modified pyrimidines (for example thiouridine and methylcytosine).

"*Stringency*" as used herein, refers to temperature and ionic strength conditions, and presence or absence of certain organic solvents and/or detergents during hybridisation. The higher the stringency, the higher will be the required level of complementarity between hybridising nucleotide sequences.

"*Stringent conditions* " designates those conditions under which only nucleic acid having a high frequency of complementary bases will hybridise.

Reference herein to low stringency conditions includes and encompasses:-
(i) from at least about 1% v/v to at least about 15% v/v formamide and from at least about 1 M to at least about 2 M salt for hybridisation at 42 °C, and at least about 1 M to at least about 2 M salt for washing at 42 °C; and
(ii) 1% Bovine Serum Albumin (BSA), 1 mM EDTA, 0.5 M NaHPO₄ (pH 7.2), 7% SDS for hybridization at 65 °C, and (i) 2xSSC, 0.1% SDS; or (ii) 0.5% BSA, 1 mM EDTA, 40 mM NaHPO₄ (pH 7.2), 5% SDS for washing at room temperature.

Medium stringency conditions include and encompass:-
(i) from at least about 16% v/v to at least about 30% v/v formamide and from at least about 0.5 M to at least about 0.9 M salt for hybridisation at 42 °C, and at least about 0.5 M to at least about 0.9 M salt for washing at 42 °C; and
(ii) 1% Bovine Serum Albumin (BSA), 1 mM EDTA, 0.5 M NaHPO₄ (pH 7.2), 7% SDS for hybridization at 65 °C and (a) 2 x SSC, 0.1% SDS; or (b) 0.5% BSA, 1 mM EDTA, 40 mM NaHPO₄ (pH 7.2), 5% SDS for washing at 42 °C.

High stringency conditions include and encompass:-
(i) from at least about 31% v/v to at least about 50% v/v formamide and from at least about 0.01 M to at least about 0.15 M salt for hybridisation at 42 °C, and at least about 0.01 M to at least about 0.15 M salt for washing at 42°C;
(ii) 1% BSA, 1 mM EDTA, 0.5 M NaHPO₄ (pH 7.2), 7% SDS for hybridization at 65 °C, and (a) 0.1 x SSC, 0.1% SDS; or (b) 0.5% BSA, 1mM EDTA, 40 mM NaHPO₄ (pH 7.2), 1% SDS for washing at a temperature in excess of 65 °C for *ca.* one hour; and
(iii) 0.2 x SSC, 0.1% SDS for washing at or above 68 °C for *ca.* 20 minutes.

Notwithstanding the above, stringent conditions are well known in the art, such as described in Chapters 2.9 and 2.10 of Ausubel *et al*., *supra.* A skilled addressee will also recognize that various factors can be manipulated to optimise the specificity of the hybridisation. Optimisation of the stringency of the final washes can serve to ensure a high degree of hybridisation.

Typically, complementary nucleotide sequences are identified by blotting techniques that include a step whereby nucleotides are immobilized on a matrix, preferably a synthetic membrane such as nitrocellulose, a hybridisation step, and a detection step. Southern blotting is used to identify a complementary DNA sequence; northern blotting is used to identify a complementary RNA sequence. Dot blotting and slot blotting can be used to identify complementary DNA/DNA, DNA/RNA or RNA/RNA polynucleotide sequences. Such techniques are well known by those skilled in the art, and have been described in Ausubel *et al., supra,* at pages 2.9.1 through 2.9.20.

According to such methods, Southern blotting involves separating DNA molecules according to size by gel electrophoresis, transferring the size-separated DNA to a synthetic membrane, and hybridising the membrane bound DNA to a complementary nucleotide sequence.

In dot blotting and slot blotting, DNA samples are directly applied to a synthetic.membrane prior to hybridisation as above.

An alternative blotting step is used when identifying complementary nucleic acids in a cDNA or genomic DNA library, such as through the process of plaque or colony hybridisation. Other typical examples of this procedure is described in Chapters 8-12 of Sambrook *et al., supra.*

Typically, the following general procedure can be used to determine hybridisation conditions. Nucleic acids are blotted/transferred to a synthetic membrane, as described above. A wild type nucleotide sequence of the invention is labelled as described above, and the ability of this labelled nucleic acid to hybridise with an immobilized nucleotide sequence analysed.

Methods for detecting labelled nucleic acids hybridised to an immobilized nucleic acid are well known to practitioners in the art. Such methods include autoradiography, chemiluminescent, fluorescent and colorimetric detection.

In another embodiment, homologous and/or variant promoter nucleic acids may be prepared according to the following procedure:
(i) obtaining a nucleic acid extract from a suitable host;
(ii) creating primers which are, optionally, degenerate wherein each comprises a respective portion of a nucleotide sequence according to SEQ ID NO:1 or SEQ ID NO:2; and
(iii) using said primers to amplify, via nucleic acid amplification techniques, one or more amplification products from said nucleic acid extract.

Examples of suitable hosts include strains of *E*. *coli* and *S. enterica* and/or species of bacteria other than *E*. *coli* and *S*. *enterica* that may have allelic variants of the promoters set forth in SEQ ID NO:1 and/or SEQ ID NO:2.

Suitable nucleic acid amplification techniques are well known to the skilled addressee, and include polymerase chain reaction (PCR) and ligase chain reaction (LCR) as for example described in Chapter 15 of Ausubel *et al*. *supra*; strand displacement amplification (SDA) as for example described in U.S. Patent No 5,422,252; rolling circle replication (RCR) as for example described in Liu et al., 1996, J. Am. Chem. Soc. 118 1587 and International application WO 92/01813, and Lizardi *et al.,* (International Application WO 97/19193); nucleic acid sequence-based amplification (NASBA) as for example described by Sooknanan et al., 1994, Biotechniques 17 1077,; ligase chain reaction (LCR) as for example described in International Application WO89/09385; and Q-β replicase amplification as for example described by Tyagi et al., 1996, Proc. Natl. Acad. Sci. USA 93 5395,; and helicase-dependent amplification as for example described in International Publication WO 2004/02025.

As used herein, an "*amplification product*" refers to a nucleic acid product generated by nucleic acid amplification techniques.

### Expression vectors and expression constructs

The invention provides genetic constructs comprising a promoter, variant or homolog of the invention which may be used to express proteins including enzymes, proteins related to the immune system, immunogenic and/or antigenic proteins, oxygen transport and storage proteins, hormones, toxins, viruses, genes and their regulation, cancer related proteins, proteins related to food poisoning, plants, signalling, movement, drug targets, protein folding, cell regulation and apoptosis, metal or ligand transport, cytokines, tumour antigens, although without limitation thereto.

Such genetic constructs are referred to herein as "*expression constructs*"*.*

In another embodiment, the invention provides a genetic construct in the form of an "*expression vector*"*,* which is a genetic construct comprising a promoter, variant or homolog of the invention, into which may be cloned, ligated or otherwise inserted a heterologous nucleic acid to thereby form an expression construct.

By "*heterologous nucleic acid*" is meant a nucleic acid distinct from an *sspA* nucleic acid or SSPA protein-encoding nucleic acid. The heterologous nucleic acid may encode one or more of the proteins hereinbefore described, although without limitation thereto.

Suitably, the heterologous nucleic acid is operably linked to the *sspA* promoter in the expression vector to form the expression construct.

By "*operably linked*" is meant that the promoter is/are positioned relative to the heterologous nucleic acid to initiate, regulate, control or otherwise facilitate transcription.

In one embodiment, the promoter is a *Salmonella sspA* promoter.

In another embodiment, the promoter is an *E. coli sspA* promoter.

In particular embodiments, the *sspΛ* promoter has a nucleotide sequence as set forth in SEQ ID NO: 1 or SEQ ID NO: 2.

In yet another embodiment, the *sspΛ* promoter is homologous to or is a fragment or a variant of either of SEQ ID NO: 1 or SEQ ID NO: 2.

It will be appreciated that expression constructs of the invention may facilitate protein expression for the more general purposes of protein purification.

However, in particular embodiments, expression of proteins may be for the purposes of prophylactic and/or therapeutic treatment of disease conditions.

Particularly contemplated is expression of immunogenic proteins which may be expressed to elicit an immune response, eg. immunogenic protein C fragment of tetanus toxin. Non-limiting examples of other immunogenic proteins of interest include bacterial antigens such as outer membrane proteins from *Haemophilus* or *Neisseria* species, viral proteins such as HIV envelope proteins, antigens and parasite antigens.

Expression vectors and constructs of the invention may be self-replicating extra-chromosomal vectors such as a plasmid, or a vector that integrates into a bacterial host chromosome. It will be appreciated by the skilled addressee that the bacterial host may be selected from a strain of *Salmonella* sp., *E.coli* or *Shigella* sp.

Expression vectors and constructs of the invention may include one or more other regulatory components in addition to said promoter.

Typically, said one or more regulatory components may include, but are not limited to, nucleotide sequences corresponding to recombination sites, leader or signal sequences, ribosomal binding sites, transcriptional start and termination sequences, translational start and termination sequences, single or multiple cloning sites and enhancer or activator sequences.

For the purposes of chromosomal integration, expression constructs of the invention include one or more recombination sequences.

A preferred recombination sequence is *aroD,* which facilitates recombination between *aroD* sequences in the vector and chromosomal *aroD* sequences. However, the skilled person will appreciate that any bacterial chromosomal sequence may be used to facilitate recombination, where said bacterial chromosomal sequence is not essential to bacterial viability and/or growth.

The expression vector may also include a selectable marker gene to allow the selection of transformed host cells. Selectable marker genes are well known in the art and will vary with the host cell used. Preferred selectable marker genes are *bla* which confers ampicillin resistance, *sacB,* which is lethal in the presence of sucrose and *lacZ* which gives a blue colour on plates containing 5-bromo-4-chloro-3-indolyl-β-D-galactopyranoside (X-Gal).

Suitably, protein expression is achieved by introducing an expression construct of the invention into a bacterial host.

Examples of bacterial hosts include but are not limited to, *Salmonella typhimurium* such as the STM1Rif strain described in the Examples, other *Salmonella* species such as *S*. *typhi, E. coli* and *Shigella* species.

Methods for introducing expression constructs into bacteria, such as heat shock and electroporation although without limitation thereto, are well known in the art.

### Pharmaceutical compositions and vaccines

It will be appreciated that the vectors, constructs and/or bacterial hosts described herein may be useful as delivery systems for administration of heterologous nucleic acids and/or encoded proteins for therapeutic and/or prophylactic treatment of one or more disease conditions.

The invention therefore provides pharmaceutical compositions that comprise one or more of an expression constructor bacterium comprising same together with a pharmaceutically-acceptable carrier, diluent or excipient.

For example, it is contemplated that the herein described pharmaceutical compositions may find use in the treatment of diseases by expressing proteins such as enzymes, immunogenic proteins, proteins involved in oxygen transport and storage, hormones, toxins, viral proteins, gene regulators such as transcription factors, tumour antigens, plant proteins, signalling proteins, proteins involved in folding, cell apoptosis proteins, metal or ligand transport proteins, or cytokines, although without limitation thereto.

Preferred pharmaceutical compositions are "*immunotherapeutic compositions*" that provide prophylactic and/or therapeutic treatment of diseases responsive to such immunotherapy, without necessarily eliciting a protective immune response.

In a preferred embodiment, the immunotherapeutic composition is a vaccine, which elicits a protective protein immune response.

By "*pharmaceutically-acceptable carrier, diluent or excipient*" is meant a solid or liquid filler, diluent or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates and pyrogen-free water.

A useful reference describing pharmaceutically acceptable carriers, diluents and excipients is Remington's Pharmaceutical Sciences (Mack Publishing Co. N.J. USA, 1991).

Any safe route of administration may be employed for providing a patient with the composition of the invention. For example, oral, rectal, parenteral, sublingual, buccal, intravenous, intra-articular, intra-muscular, intra-dermal, subcutaneous, inhalational, intraocular, intraperitoneal, intracerebroventricular, transdermal and the like may be employed

Dosage forms include tablets, dispersions, suspensions, injections, solutions, syrups, troches, capsules, suppositories, aerosols, transdermal patches and the like-These dosage forms may also include injecting or implanting controlled releasing devices designed specifically for this purpose or other forms of implants modified to act additionally in this fashion. Controlled release of the therapeutic agent may bc effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, the controlled release may be effected by using other polymer matrices, liposomes and/or microspheres.

Pharmaceutical compositions of the present invention suitable for oral or parenteral administration may be presented as discrete units such as capsules, sachets or tablets each containing a pre-determined amount of one or more therapeutic agents of the invention, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association one or more agents as described above with the carrier which constitutes one or more necessary ingredients. In general, the compositions are prepared by uniformly and intimately admixing the agents of the invention with liquid carriers or finely divided solid carriers or both, and then, if necessary, shaping the product into the desired presentation.

The above compositions may be administered in a manner compatible with the dosage formulation, and in such amount as is pharmaceutically-effective. The dose administered to a patient, in the context of the present invention, should be sufficient to effect a beneficial response in a patient over an appropriate period of time. The quantity of agent(s) to be administered may depend on the subject to be treated inclusive of the age, sex, weight and general health condition thereof, factors that will depend on the judgement of the practitioner.

Suitably, the pharmaceutical composition is administrable to an animal host, inclusive of domestic animals, livestock, poultry, performance animals and humans.

In one embodiment, the pharmaceutical composition may be in the form of an immunotherapeutic composition tor the treatment of discase(s) responsive to immunotherapy, together with an immunologically-acceptable carrier, diluent or excipient.

The present invention provides an immunotherapeutic composition or vaccine which may be used therapeutically or prophylactically which comprise, as an active, a heterologous nucleic acid which encodes one or more of an antigenic and/or immunogenic protein.

By *"antigenic"* is meant capable of being recognized by components of the host immune system, such as antibodies.

By "*immunogenic*" is meant capable of eliciting an immune response, preferably a protective immune response upon administration to a host.

Any suitable procedure is contemplated for producing such vaccines. Exemplary procedures include, for example, those described in New Generation Vaccines (1997, Levine et al., Marcel Dekker, Inc. New York, Basel, Hong Kong).

Preferably, the vaccines of the invention are administered in the form of attenuated bacterial vaccines. Suitable attenuated bacteria include *Salmonella* species, for example *Salmonella enterica* var. Typhimurium or *Salmonella typhi.* Alternatively, other enteric pathogens such as *Shigella* species or *E*. *coli* may be used in attenuated form. Attenuated *Salmonella* strains have been constructed by inactivating genes in the aromatic amino acid biosynthetic pathway (Alderton *et al.,* Avian Diseases 35 435), by introducing mutations into two genes in the aromatic amino acid biosynthetic pathway (such as described in U.S. patent 5,770,214) or in other genes such as *htrA* (such as described in U.S. patent 5,980,907) or in genes encoding outer membrane proteins, such as *ompR* (such as described in U.S. patent 5,851,519).

The immunotherapeutic compositions and/or vaccines of the invention may include an immunologically-acceptable carrier, diluent or excipient. The "*immunologically-acceptable carrier, diluent or excipient*" includes within its scope water, bicarbonate buffer, phosphate buffered saline or saline and/or an adjuvant as is well known in the art. Suitable adjuvants include, but are not limited to, surface active substances such as hexadecylamine, octadecylamine, octadecyl amino acid esters, lysolecithin, dimethyldioctadecylammonium bromide, N,N-dicoctadecyl-N',N'bis(2-hydroxyethyl-propanediamine), methoxyhexadecylglycerol, and pluronic polyols; polyamines such as pyran, dextransulfate, poly 1C carbopol; peptides such as muramyl dipeptide and derivatives, dimethylglycine, tuftsin; oil emulsions; and mineral gels such as aluminum phosphate, aluminum hydroxide or alum; lymphokines, QuilA and immune stimulating complexes (ISCOMS).

Useful carriers are well known in the art and include, *eg*. thyroglobulin; albumins such as human serum albumin, toxins, toxoids or any mutant cross reactive material (CRM) of the toxin from tetanus, diptheria, pertussis, *Pseudomonas, E. coli, Staphylococcus,* and *Streptococcus,* polyamino acids such as poly(lysine:glutamie acid), influenza; Rotavirus VP6, Parvovirus VP1 and VP2, hepatitis B virus core protein, hepatitis B virus recombinant vaccine and the like. Alternatively, a fragment or epitope of a carrier protein or other immnogenic protein may be used. For example, a T cell epitope of a bacterial toxin, toxoid or CRM may be used. In this regard, reference may be made to U.S. Patent No 5,785,973.

The immunotherapeutic compositions and/or vaccines of the invention may be administered in multivalent form in combination with antigens of organisms inclusive of the pathogenic bacteria *H. influenzae* and other *Haemophilus* species, *M. catarrhalis, N. gonorrhoea, E. coli, S. pneumoniae, etc..*

Multivalent vaccines can be prepared wherein the heterologous nucleic acid encodes:
(i) a plurality of epitopes of an immunogenic protein;
(ii) a plurality of epitopes, each derived from a different immunogenic protein of the same organism; or
(iii) a plurality of cpitopes derived from immunogenic protein of different organisms.

Throughout this specification, unless the context required otherwise, "comprise", comprises" and "comprising" are used inclusively rather than exclusively, in that one or more other integers or groups of integers may be included with a stated integer or group of integers.

In order that the invention may be readily understood and put into practical effect, particular embodiments will now be described by way of the following non-limiting examples.

### EXAMPLE 1

### Construction of the plasmid pKKsspAtctC

The *sspA* gene has been identified and characterised in *E. coli.* The equivalent gene in *Salmonella* has not been formally characterised, however BLAST searches (Altschul et al. 1997, Nucleic Acids Res. 25:3389-3402) were used to identify a homologue to the *E. coli sspA* gene in the *Salmonella enterica* serovar *typhimurium* LT2 genome (GenBank Accession number AE008854) (Mclclland et. al., 2001, Nature 413 852-856). The *E. coli* and *S. enterica* promoter sequences were aligned (Figure 1). Oligonucleotide primers (Table 1) were designed to amplify the promoters from the *E. coli* (primers ST35, ST36) and *S*. *enterica* (ST37, ST36) genomes. Promoters were amplified directly from bacterial colonies using Platinum® Pfx DNA polymerase (Invitrogen) on an Omn-E Thermal Cycler (Hybaid) with the following program: 94°C for 2 min then 30 cycles of 94°C for 15 s, 55°C for 30 s, 68°C for 30 s. After amplification, DNA products were separated on a 2% agarose gel and a ~270 bp product corresponding to the *sspA* promoter from each bacterial species was purified from the gel using a Qiaquick gel extraction kit (Qiagen).

The amplified promoter fragments were digested with the restriction enzyme *Eco*RI for 1.5 h at 37 °C. The plasmid pKK/ppagC/tetC (Dunstan et al., 1999, Infect. Immun. 67 5133-5141) was digested with *Bam*HI. The restriction enzymes was inactivated by heating at 65 °C for 20 min and the DNA fragments were blunted-ended using T4 DNA polymerase (0.5 µl of NEB T4 DNA polymerase, 125 µM final concentration of dNTPs, 5 min at 37 °C followed by 10 min at 75 °C to inactivate the polymerase). Both the plasmids fragments and the promoter fragments were then digested with *Nco*I for 1.5 hr, and the digested products were separated on a 2 % (promoter fragment) or a 1 % (plasmid) gel and the appropriately sized bands were purified from the gel using a Qiaquick gel extraction kit (Qiagen) and ligated together, followed by transformation into DH5α *E*. *coli* cells. Colonies were screened for the correct insertion of the *sspΛ* promoters and correct plasmids were sequenced. The plasmid containing the *Salmonella sspΛ* promoter was named pKKsspAtetC and the plasmid containing the *E. coli* promoter was named pKKEsspAtetC.

### EXAMPLE 2

### Construction of plasmid pCVDaroDsspAtetC

A fragment of the 5' end of the *aroD* gene was amplified by PCR using primer ST06 which incorporates a *Xba*I site and primer ST07 which incorporates a *SphI* site using *S. enterica* strain 180*galE* as a template and using an Omn-E Thermal Cycler (hybaid) with the following program:- 95°C for 2 min then 28 cycles of 95 °C for 30 s, 50 °C for 30 s, 72 °C for 40 s and a final extension step of 72 °C for 2 min. PCR products were analysed on a 1% agarose gel and the 521 bp fragment of the *aroD* gene was extracted using a Qiaquick gel extraction kit (Qiagen). The PCR product was digested with *Xba*I and *Sph*I and the enzymes were removed using a Wizard^{™} DNA Cleanup kit (Promega). The suicide vector pCVD442 (Donnenberg & Kaper, 1991, Infect. Immun. 59 4310-4317) was similarly digested with *Sph*I and XbaI, treated with shrimp alkaline phosphatase (USB) and applied to a 0.8 % agarose gel. The linearised vector was extracted from the gel using a Qiaquick gel extraction kit (Qiagen) and ligated to the digested *aroD* PCR product. Ligations were transformed by electroporation into *E. coli* DH5αλ*pir* cells. The resulting transformants contained plasmid pCVDaroD.

Plasmid pCVDaroDins was constructed by amplifying ~500 bp from the C-terminus of the *S. enterica* strain STMI *aroD* gene by PCR using primers ST10 and ST11 (Table 1) that introduce *Sac*I and *Eco*RV restriction sites respectively at either end of the PCR product. Digestion of the PCR product with *Sac*I and *Eco*RV and plasmid pCVDaroD with *Sac*I and *Sma*I followed by ligation and transformation results in the construction of plasmid pCVDaroDins that includes two sections of the *aroD* gene separated by *Sac*I*, Not*I and *Sph*I restriction sites. Antigens and promoters can be cloned into these sites either by restriction digestion of purified plasmids or by digestion of PCR products synthesised with appropriately designed primers followed by ligation to digested pCVDaroDins vector.

The promoter-*tet*C gene cassettes from plasmids pKKsspAtetC and pKK/p*pagC*/*C* frag were amplified using oligonucleotide primers (ST37 and ST43 for pKKsspAtctC and ST44 and ST43 for pKK/*pagC*/*C* frag and *PfuTurbo*® DNA polymerase (Stratagene), digested with *Sac*I and *Sph*I and ligated to pCVDaroDins, which had been digested similarly. The resulting transformants contained the constructs pCVDaroDsspAtetC and pCVDaroDpagCtetC.

### EXAMPLE 3

### Development of Rifampicin resistant Salmonella enterica strain STM1

A colony of *S. enterica* strain STM1 was inoculated into 20 ml of LB broth and grown at 37°C with agitation for 9 hr. Serial dilutions from the culture were plated on LB agar + rifampicin (50 µg/ml) and grown overnight at 37°C. Rifampicin-resistant colonies were subcultured onto LB-rifampicin plates and after a second overnight growth the pure cultures of STM1/Rif were stored at -70 °C with 20 % glycerol. Lipopolysaccharide (LPS) profiles of the strains were checked using standard techniques to confirm that smooth LPS were still expressed by STM1/Rif (Apicella et al, 1994, Meth. Enzymol. 235 242-252).

### EXAMPLE 4

### Construction of STM1/Rif with chromosomally integrated pCVDaroDsspAtetC and pCVDaroDpagCtetC

In order to construct bacterial strains in which plasmids are integrated in the STM1/Rif chromosome, plasmids pCVDaroDsspAtetC and pCVDaroDpagCtetC were transformed into the *E*. *coli* conjugative donor strain S17.1λ*pir* by electroporation. Cultures of S17.1λ*pir* cells harbouring the plasmid were cross-streaked onto an LB agar plate with a *S*. *enterica* STM1/Rif culture. Plates were incubated at 37°C for 8 h before the cultures were harvested into LB broth and serial dilutions of each conjugation plated on LB agar plates containing Ampicillin (50 µg/ml), rifampicin (50 µg/ml) and 10 ml/L Aromix (4 mg/ml phenylalanine, 4 mg/ml tyrosine, 4 mg/ml tryptophan, 1 mg/ml 2,3 dihydroxybenzoate, 1 mg/ml p-aminobenzoic acid) and grown overnight at 37 °C. Transconjugants were screened for expression of fragment C and the presence of the promoter-*tet*C construct on the STM1/Rif chromosome was confirmed by PCR using either primer ST22 or ST23 (which bind outside the section of the *aroD* gene cloned in pCVDaroDins) and a primer that binds within the antigen cassette. A strain was identified that had the whole of plasmid pCVDaroDsspAtetC (Strain JE01) or pCVDaroDpagCtetC (Strain QE08) integrated into the *aroD* gene of the STM1/Rif chromosome.

Plasmids derived from pCVD442 such as pCVDaroDsspAtetC include the *SacB* gene from *Bacillus subtilis,* which is toxic to *Salmonella* in the presence of sucrose. Hence, growth of *Salmonella* with a chromosomally integrated pCVD442 derivate in the presence of sucrose can be used to select for the loss of the chromosomally integrated plasmid through recombination. Sucrose resistant colonies derived from strain JE01 have either lost the entire plasmid, including the *sspA-tetC* cassette (reforming the wild-type *aroD* gene), or alternatively the plasmid backbone is lost leaving just the *sspA-tetC* cassette integrated in the *aroD* gene in the chromosome (a double-crossover). All the sucrose resistant colonies isolated when strain JE01 was grown up in LB Broth and then plated at a range of dilutions of LB agar plates that lacked NaCl but contained 20 % sucrose and Aromix followed by growth at 28°C for 24-36 h did not contain the *sspA-tetC* cassette.

An alternative approach, using blue/white screening was used to create a *S. enterica* strain with only the *sspA-tetC* cassette in the *aroD* gene. First, the *lacZ* gene from *E. coli* encoding the enzyme β-galactosidase was cloned into plasmid pKKsspAtetC, replacing the *tetC* gene and creating plasmid pKKsspAlacZ. The *sapA-lacZ* cassette was transferred to plasmid pCVDaroDins, conjugated into STM1/Rif and, sucrose resistant colonies were isolated as described above. Sucrose resistant colonies were plated on media containing 40 µg/ml 5-bromo-4-chloro-3-indolyl-β-D-galactopyranoside (X-Gal). Sucrose resistant colonies that were blue in colour had retained the *sspA-lacZ* cassette but lost the plasmid backbone. One strain, QD03, was used to create an *sspA-tetC* double crossover. The pCVDaroDsspAtetC plasmid was introduced into strain QD03 by conjugation, resulting in recombination in the *aroD-sspA-lacZ,* allele. After selection on LB (no NaCl) 20 % sucrose plates containing 40 µg/ml X-Gal, a few white colonies amongst thousands of blue colonies were observed. These white colonies were characterised by PCR and found to contain the *sspA-tetC* cassette in the *aroD* gene and express fragment C, but had lost the pCVDaroDsspAtetC vector backbone and the *lacZ* gene. One strain was designated JD09 and used in further experiments.

### EXAMPLE 5

### Expression of tetanus toxoid fragment C in vitro

Fragment C expression was examined in STM1/Rif strains harbouring chromosomally integrated pCVDaroDsspATetC (JE01), the *sspA-tetC* cassette chromosomally as a double crossover (JD09), plasmid pKKsspAtetC (JD06) and plasmid pKK/ppagC/C frag (JB07). A colony of each strain was inoculated into 3 ml of LB broth supplemented with Ampicillin (100 g/ml) where appropriate and grown overnight at 37 °C. One ml of this culture was inoculated into 20 ml of LB broth in a 50 ml Falcon tube supplemented with Ampicillin (100 µg/ml) where appropriate. Cells were grown to an OD at 600 nm of 0.5, harvested, washed in PBS and resuspended at an A₆₀₀ nm of 10. Cells were disrupted by sonication and cell debris pelleted by centrifugation at 13,000 x g for 5 min. The concentration of total cell protein in the supernatant was determined using a BCA Protein Assay (Pierce) and concentrations of total cell protein adjusted so that all samples were identical. Serial dilutions of the supernatant were applied to nitrocellulose membrane, and the fragment C protein detected using monoclonal anti-tetanus toxin C fragment antibody (Roche) and goat anti-mouse IgG alkaline phosphatase conjugated secondary antibody. Both chromosomal and plasmid encoded fragment C construct produced significant amounts of TetC protein (FIG. 3).

### EXAMPLE 6

### Immune responses in BALB/c mice inoculated with STM1/Rif strains with plasmid or chromosomally encoded tetanus toxoid fragment C

Master vaccine banks were prepared by growing each strain overnight in 5 ml of LB broth, supplemented with 100 µg/ml Ampicillin where appropriate, at 37 °C. The overnight cultures were used to inoculate 300 ml cultures of LB broth, supplemented with 100 µg/ml Ampicillin where appropriate, which were grown to an OD at 600 nm of 0.5 at 37 °C with vigourous shaking. Cultures were rapidly cooled in an ice-water bath. Vaccine banks were prepared by adding 40 ml of culture to 10 ml of sterile glycerol in each of 6 50 ml Falcon tubes. The cells were rapidly frozen in an ethanol/dry ice bath and stored at-80 °C,

To prepare vaccines, one tube of each vaccine bank was defrosted in a 37 °C waterbath and added to 300 ml of pre-warmed LB broth in a 1 litre flask, supplemented with 100 µg/ml Ampicillin where appropriate. Cultures were grown at 37°C with vigorous shaking to an OD at 600 nm of 0.5. Cultures were chilled for 5 minutes on ice, before the cells were harvested by centrifugation, and washed twice with PBS. After the final wash, cells were re-suspended in 1 ml of PBS and the concentration adjusted to 20 OD_{600 nm}/ml (1 OD_{600 nm}/ml = 5 x 10⁸ cfu/ml). Immediately before vaccination, the cells were mixed with an equal volume of 3 % sodium bicarbonate.

Groups of 7, 6-8 week old BALB/c mice were orally inoculated with 0.2 ml of *Salmonella*/*sodium* bicarbonate solution after 2-3 hr without fluids. Mice were vaccinated on day 0, 14 and 21 and sera were collected on day 0, day 35 and day 49.

Serum antibody responses against fragment C were quantified using a standard end-point ELISA using tetanus toxoid (Dunstan *et al.,* 1999, *supra*). Strain JD06 containing plasmid pKKsspAtetC produced excellent immune responses (FIG 4) with no statistical difference compared to strain JB07 containing plasmid pKK/p*p*ag*C*/frag C. In another experiment responses against fragment C elicited by strain JD06 utilizing the *Salmonella sspA* promoter (average titre 22116) were not significantly different (unpaired t-test, p=0.29) to those elicited by strain PC01 containing utilizing the *E*. *coli sspA* promoter (average titre 48205).

The *sspA* promoter was also able to generate immune responses (FIG 5) in some mice as a chromosomal insertion, both as single crossover (strain JE01, pCVD442 vector backbone in the chromosome) and as a double crossover (JD09). In contrast, strain QE08 which is identical to strain JE01 except that the promoter driving expression was *pagC* rather than *sspA,* did not produce any immune responses (FIG. 6).

**TABLE 1**

| **SEQ ID** | **Name** | **Oligonucleotide Sequence (5'-3')** | **Target** | **Enzymes included** |
|---|---|---|---|---|
| SEQ ID NO:3 | ST06 | GACATCTAGAGGTACCAAATGAAAACCGT | *S*. *enterica aroD* | *Xba*I |
| SEQ ID NO:4 | ST07 | ATATCGCAIGCCAGTTCCTGCATTTTC | *S. enterica aroD* | *Sph*I |
| SEQ ID NO:5 | ST10 | ATGGAGCTCGCGGCCGGTGATATTCCGAAG | *S*. *enterica aroD* | *Sac*I, *Not*I |
| SEQ ID NO:6 | ST11 | GGCGATATCTGGAATATAATITACTG | *S. enterica aroD* | *Eco*RV |
| SEQ ID NO:7 | ST22 | GTTATGGCAAGGAGCCGA | 5' end *S. enterica aroD* | - |
| SEQ ID NO:8 | ST23 | GATAAATCTCGTGGTGAC | 3' end *S*. *enterica aroD* | - |
| SEQ ID NO:9 | ST35 | ATCGGAATTCGAGCTCTAAGTCAACTATTTCAGACT | *E. coli sspA* promoter 5' end | *Eco*RI, *Sac*I |
| SEQ ID NO:10 | ST36 | GCAGGGATCCCCATGGAAACCTCCACGTAT | *E. coli* and *S*. *enterica sspA* promoter 3'end | *Bam*HI, *Nco*I |
| SEQ ID NO:11 | ST37 | GACAGAATTCGAGCTCGGGCGAGCATTTCAGACC | *S. enterica sspA* promoter 5' end | *Eco*RI, *Sac*I |
| SEQ ID NO.12 | ST43 | GCAGGCATGCGTCTCATGAGCGGATACA | 3' of *rrnB* terminator in pKKsspAtetC | *Sph*I |
| SEQ ID NO:13 | ST44 | GCAGGAGCTCGTAGAGGATCCTAATGGGT | 5' end of *pagC* promoter | Sad |

**TABLE 2**

| **Plasmid or strain** | **Description** | **Reference** |
|---|---|---|
| DH5α | General cloning strain of *E*. *coli* | Woodcock et al, 1989, Nucl. Acids Res 17 3469-3478 |
| DH5αλ*pir* | Cloning strain with *pir* lambda lysogen, host for pCVD442 | Gift from M.S. Donnenberg |
| S17.1λ*pir* | Conjugative donor strain, host for pCVD442 | Simon et al., 1983, Bio/Technology 1784-791 |
| STM1 | *Salmonella enterica* serovar *typhimurium aroA-* | Alderton et al., 1991, Avian Diseases 35 435-442 |
| JA08 | Rifampicin resistant STM1 | - |
| JB07 | JA08 + pKK/p*pagC*/*C* frag | - |
| JD06 | JA08 + pKKsspAtetC | - |
| PC01 | JA08 + pKKsspAEtetC | - |
| JD09 | JA08/*sspA-tetC* (double crossover, no vector backbone remaining) | - |
| JE01 | JA08/chromosomal pCVDaroDsspAtetC | - |
| QD03 | JA08/*sspA-lacZ* (double crossover, no vector backbone remaining) | - |
| QE08 | JA08/chromosomal pCVDaroDpagCtetC | - |
| pCVD442 | Suicide vector, requires *pir* for replication, contains *sacB* gene | Donnenberg & Kaper,1991, Infect. Immun. 594310-4317 |
| PCVDaroD | PCVD442 with 5' section of *S. enterica aroD* gene | - |
| PCVDacoDins | PCVD442 with 5' and 3' sections of *S. enterica aroD* gene with *Sac*I*, Not*I and *Sph*I cloning sites in the centre | - |
| pKk/p*pagC*/*C* frag | Plasmid with *pag*C promoter expressing fragment C of tetanus toxin | Dunstan et al., 1999, Infect Immun 67 5133-5141 |
| pKKsspAtetC | Plasmid with *Salmonella sspA* promoter expressing fragment C of tetanus toxin | - |
| pKKEsspAtetC | Plasmid with *E. coli sspA* promoter expressing fragment C of tetanus toxin | - |
| pCVDaroDsspAtetC | PCVDaroDins with *sspA-tetC* in the middle of *aroD* | - |
| pKKsspAlacZ | Plasmid with *Salmonella sspA* promoter expressing *lacZ* | - |
| pCVDaroDpagCtetC | PCVDaroDins with *pagC-tetC* in the middle of *aroD* | - |

## Claims

1. An isolated nucleic acid comprising at least one promoter having a nucleotide sequence set forth in SEQ ID NO: 1, wherein the at least one promoter is capable of controlling the expression of a heterologous nucleic acid.

2. An isolated nucleic acid comprising at least one promoter active fragment of the nucleotide sequence set forth in SEQ ID NO: 1, wherein said promoter active fragment has at least 50% of the activity of a regulatable promoter comprising the nucleotide sequence of SEQ ID NO:1 and wherein the at least one promoter active fragment is capable of controlling the expression of a heterologous nucleic acid.

3. An isolated nucleic acid comprising a regulatable promoter or promoter active fragment thereof having at least 60 % nucleotide sequence identity with the nucleotide sequence set forth in SEQ ID NO: 1, but excluding the nucleotide sequence set forth in SEQ ID NO: 2, wherein said promoter active fragment has at least 50% of the activity of a regulatable promoter comprising the nucleotide sequence of SEQ ID NO:1 and wherein the at least one promoter or promoter active fragment is capable of controlling the expression of a heterologous nucleic acid.

4. A chimeric gene comprising, the isolated nucleic acid of Claim 3, or a promoter active fragment thereof, and a heterologous nucleic acid.

5. An expression vector comprising the isolated nucleic acid of any one of claims 1-3.

6. The expression vector of claim 5 wherein said expression vector is maintainable extra-chromosomally in a bacterium.

7. The expression vector of claim 5 wherein said expression vector is chromosomally integratable and maintainable in a bacterium.

8. The expression vector of claim 5 wherein said promoter is a *Salmonella sspA* promoter and the bacterium is of a *Salmonella* strain.

9. An expression construct comprising the expression vector of any one of claims 5-8 and a heterologous nucleic acid operably linked to said promoter.

10. A bacterium transformed with the expression construct of claim 9.

11. The bacterium of claim 10 wherein said bacterium is of a strain of *Salmonella* sp.

12. The bacterium of claim 10 wherein said expression construct is chromosomally-integrated and maintained in said bacterium.

13. The bacterium of claim 10 wherein said expression construct is maintained extra-chromosomally in said bacterium.

14. The bacterium of claim 10, which is attenuated.

15. An immunotherapeutic composition comprising an expression construct that comprises a promoter of bacterial origin which is regulatable by starvation conditions and comprises: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2; which is operably linked to a heterologous nucleic acid encoding an immunogenic protein.

16. The immunotherapeutic composition of claim 15 which is a vaccine.

17. An immunotherapeutic composition comprising a bacterium transformed with an expression construct having a promoter of bacterial origin which is regulatable by starvation conditions and comprises: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2; which is operably linked to a heterologous nucleic acid encoding an immunogenic protein, together with a pharmaceutically-acceptable carrier, diluent or excipient.

18. The immunotherapeutic composition of claim 17 wherein said bacterium is attenuated.

19. The immunotherapeutic composition of claim 17 wherein said bacterium is of a strain of *Salmonella* sp.

20. The immunotherapeutic composition of claim 17 wherein said expression construct is chromosomally-integrated and maintained in said bacterium.

21. The immunotherapeutic composition of claim 17 wherein said expression construct is maintained extra-chromosomally in said bacterium.

22. The immunotherapeutic composition of claim 17, which is a vaccine.

23. The use of an expression construct having a promoter of bacterial origin regulatable by starvation conditions and comprising: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2; operably linked to a heterologous nucleic acid that encodes an immunogenic protein suitable for therapy and/or prophylaxis of one or more disease conditions responsive to immunotherapy in a host, for the preparation of a medicament for the treatment and/or prophylaxis of one or more disease conditions.

24. The use of an expression construct having a promoter of bacterial origin regulatable by starvation conditions and comprising: a nucleotide sequence set forth in SEQ ID NO: 1 or SEQ ID NO: 2; at least a promoter active fragment of SEQ ID NO: 1 or SEQ ID NO: 2; or a nucleotide sequence at least 60% identical to SEQ ID NO:1 or SEQ ID NO:2 operably linked to a heterologous nucleic acid that encodes an immunogenic protein, for the preparation of a medicament for vaccinating a host.

25. The use of Claim 24 wherein a protective immune response is induced in said host.

26. The use of any one of claims 23 to 25, wherein the expression construct is present in an attenuated bacterium.

27. The use of Claim 26, wherein the bacterium is a strain of *Salmonella sp.*

28. The use according to any one of claims 23 to 25 wherein said host is a human.

## Patentansprüche

1. Isolierte Nukleinsäure, umfassend zumindest einen Promotor mit der in SEQ ID NO:1 gezeigten Nukleotidsequenz, wobei der zumindest eine Promotor die Expression einer heterologen Nukleinsäure kontrollieren kann.

2. Isolierte Nukleinsäure, umfassend zumindest ein aktives Promotorfragment der in SEQ ID NO:1 gezeigten Nukleotidsequenz, wobei das aktive Promoterfragment mindestens 50% der Aktivität eines regulierbaren Promotors hat, der die Nukleotidsequenz von SEQ ID NO:1 umfasst, und wobei der zumindest ein aktives Promotorfragment die Expression einer heterologen Nukleinsäure kontrollieren kann.

3. Isolierte Nukleinsäure, umfassend einen regulierbaren Promotor oder ein aktives Promotorfragment davon mit mindestens 60% Nukleotidsequenzidentität mit der in SEQ ID NO:1 gezeigten Nukleotidsequenz, ausschließlich der in SEQ ID NO:2 gezeigten Nukleotidsequenz, wobei das aktive Promotorfragment mindestens 50% der Aktivität eines regulierbaren Promotors hat, der die Nukleotidsequenz von SEQ ID NO:1 umfasst, und wobei der zumindest ein Promotor oder ein aktives Promotorfragment die Expression einer heterologen Nukleinsäure kontrollieren kann.

4. Chimäres Gen, umfassend die isolierte Nukleinsäure von Anspruch 3 oder ein aktives Promotorfragment davon und eine heterologe Nukleinsäure.

5. Expressionsvektor, umfassend die isolierte Nukleinsäure nach einem der Ansprüche 1 bis 3.

6. Expressionsvektor nach Anspruch 5, wobei der Expressionsvektor in einem Bakterium extrachromosomal haltbar ist.

7. Expressionsvektor nach Anspruch 5, wobei der Expressionsvektor in einem Bakterium chromosomal integrierbar und haltbar ist.

8. Expressionsvektor nach Anspruch 5, wobei der Promotor ein *Salmonella sspA-*Promotor ist und das Bakterium von einem *Salmonella*-Stamm ist.

9. Expressionskonstrukt, umfassend den Expressionsvektor nach einem der Ansprüche 5 bis 8 und eine heterologe Nukleinsäure, die funktionell mit dem Promotor verbunden ist.

10. Bakterium, transformiert mit dem Expressionskonstrukt nach Anspruch 9.

11. Bakterium nach Anspruch 10, wobei das Bakterium von einem Stamm von *Salmonella* sp. ist.

12. Bakterium nach Anspruch 10, wobei das Expressionskonstrukt in dem Bakterium chromosomal integriert ist und beibehalten wird.

13. Bakterium nach Anspruch 10, wobei das Expressionskonstrukt in dem Bakterium extrachromosomal beibehalten wird.

14. Bakterium nach Anspruch 10, das attenuiert ist.

15. Immuntherapeutische Zusammensetzung, umfassend ein Expressionskonstrukt, das einen Promotor bakteriellen Ursprungs umfasst, der unter Hungerzuständen regulierbar ist und umfasst: eine in SEQ ID NO:1 oder SEQ ID NO:2 gezeigte Nukleotidsequenz; zumindest ein aktives Promotorfragment der SEQ ID NO:1 oder SEQ ID NO:2; oder eine zu SEQ ID NO:1 oder SEQ ID NO:2 mindestens 60% identische Nukleotidsequenz; der mit einer heterologen Nukleinsäure funktionell verbunden ist, die ein immunogenes Protein kodiert.

16. Immuntherapeutische Zusammensetzung nach Anspruch 15, die ein Vakzin ist.

17. Immuntherapeutische Zusammensetzung, umfassend ein Bakterium, transformiert mit einem Expressionskonstrukt, das einen Promotor bakteriellen Ursprungs hat, der unter Hungerzuständen regulierbar ist und umfasst: eine in SEQ ID NO:1 oder SEQ ID NO:2 gezeigte Nukleotidsequenz; zumindest ein aktives Promotorfragment der SEQ ID NO:1 oder SEQ ID NO:2; oder eine zu SEQ ID NO:1 oder SEQ ID NO:2 mindestens 60% identische Nukleotidsequenz; der mit einer heterologen Nukleinsäure funktionell verbunden ist, die ein immunogenes Protein kodiert, zusammen mit einem pharmazeutisch verträglichen Träger, Verdünnungsmittel oder Exzipient.

18. Immuntherapeutische Zusammensetzung nach Anspruch 17, wobei das Bakterium attenuiert ist.

19. Immuntherapeutische Zusammensetzung nach Anspruch 17, wobei das Bakterium von einem Stamm von *Salmonella* sp. ist.

20. Immuntherapeutische Zusammensetzung nach Anspruch 17, wobei das Expressionskonstrukt in dem Bakterium chromosomal integriert ist und beibehalten wird.

21. Immuntherapeutische Zusammensetzung nach Anspruch 17, wobei das Expressionskonstrukt in dem Bakterium extrachromosomal beibehalten wird.

22. Immuntherapeutische Zusammensetzung nach Anspruch 17, die ein Vakzin ist.

23. Verwendung eines Expressionskonstrukts, das einen Promotor bakteriellen Ursprungs hat, der unter Hungerzuständen regulierbar ist und aufweist: eine in SEQ ID NO:1 oder SEQ ID NO:2 gezeigte Nukleotidsequenz; zumindest ein aktives Promotorfragment der SEQ ID NO:1 oder SEQ ID NO:2; oder eine zu SEQ ID NO:1 oder SEQ ID NO:2 mindestens 60% identische Nukleotidsequenz; der mit einer heterologen Nukleinsäure funktionell verbunden ist, die ein für die Therapie und/oder Vorbeugung eines oder mehrerer Krankheitszustands/Krankeitszustände nützliches immunogenes Protein kodiert, zur Herstellung eines Arzneimittels für die Behandlung und/oder Vorbeugung eines oder mehrerer Krankheitszustands/Krankheitszuständen.

24. Verwendung eines Expressionskonstrukts, das einen Promotor bakteriellen Ursprungs hat, der unter Hungerzuständen regulierbar ist und aufweist: eine in SEQ ID NO:1 oder SEQ ID NO:2 gezeigte Nukleotidsequenz; zumindest ein aktives Promotorfragment der SEQ ID NO:1 oder SEQ ID NO:2; oder eine zu SEQ ID NO:1 oder SEQ ID NO:2 mindestens 60% identische Nukleotidsequenz; der mit einer heterologen Nukleinsäure funktionell verbunden ist, die ein immunogenes Protein kodiert, für die Herstellung eines Medikaments für die Vakzinierung eines Wirts.

25. Verwendung nach Anspruch 24, wobei in dem Wirt eine schützende Immunantwort induziert wird.

26. Verwendung nach einem der Ansprüche 23 bis 25, wobei das Expressionskonstrukt in einem attenuierten Bakterium vorhanden ist.

27. Verwendung nach Anspruch 26, wobei das Bakterium von einem Stamm von *Salmonella* sp. ist.

28. Verwendung nach einem der Ansprüche 23 bis 25, wobei der Wirt ein Mensch ist.

## Revendications

1. Acide nucléique isolé, comprenant au moins un promoteur ayant une séquence de nucléotides illustrée dans la SEQ ID N° : 1, dans lequel l'au moins un promoteur est apte à contrôler l'expression d'un acide nucléique hétérologue.

2. Acide nucléique isolé, comprenant au moins un fragment actif de promoteur correspondant à la séquence de nucléotides illustrée dans la SEQ ID N° : 1, dans lequel ledit fragment actif de promoteur a au moins 50 % de l'activité d'un promoteur régulable comprenant la séquence de nucléotides correspondant à la SEQ ID N° : 1 ; et dans lequel l'au moins un fragment actif de promoteur est apte à contrôler l'expression d'un acide nucléique hétérologue.

3. Acide nucléique isolé, comprenant un promoteur ou un fragment actif de ce promoteur, régulable, ayant une identité de séquence de nucléotides d'au moins 60 % avec la séquence de nucléotides illustrée dans la SEQ ID N° : 1, mais à l'exclusion de la séquence de nucléotides illustrée dans la SEQ ID N° : 2, dans lequel ledit fragment actif de promoteur a au moins 50 % de l'activité d'un promoteur régulable comprenant la séquence de nucléotides correspondant à la SEQ ID N° : 1, et dans lequel l'au moins un promoteur ou fragment actif de ce promoteur est apte à contrôler l'expression d'un acide nucléique hétérologue.

4. Gène chimérique comprenant l'acide nucléique isolé de la revendication 3 ou un fragment actif de promoteur de celui-ci, et un acide nucléique hétérologue.

5. Vecteur d'expression, comprenant l'acide nucléique isolé de l'une quelconque des revendications 1 à 3.

6. Le vecteur d'expression de la revendication 5, ledit vecteur d'expression étant apte à une conservation extrachromosomique dans une bactérie.

7. Le vecteur d'expression de la revendication 5, ledit vecteur d'expression étant apte à une intégration et une conservation chromosomiques dans une bactérie.

8. Le vecteur d'expression de la revendication 5, dans lequel ledit promoteur est un promoteur de *Salmonella ssp A,* et la bactérie est d'une souche de *Salmonella.*

9. Construction d'expression, comprenant le vecteur d'expression de l'une quelconque des revendications 5 à 8, et un acide nucléique hétérologue fonctionnellement lié au dit promoteur.

10. Bactérie transformée avec la construction d'expression de la revendication 9.

11. La bactérie de la revendication 10, ladite bactérie étant d'une souche de *Salmonella sp.*

12. La bactérie de la revendication 10, dans laquelle ladite construction d'expression est intégrée et conservée dans un chromosome de ladite bactérie.

13. La bactérie de la revendication 10, dans laquelle ladite construction d'expression est conservée à l'extérieur d'un chromosome dans ladite bactérie.

14. La bactérie de la revendication 10, qui est atténuée.

15. Composition immunothérapeutique comprenant une construction d'expression, qui contient un promoteur d'origine bactérienne, qui est régulable par des conditions d'inanition, et comprend : une séquence de nucléotides illustrée dans la SEQ ID N° : 1 ou la SEQ ID N° : 2 ; au moins un fragment actif de promoteur correspondant à la SEQ ID N° : 1 ou la SEQ ID N° : 2 ; ou une séquence de nucléotides au moins 60 % identique à la SEQ ID N° : 1 ou la SEQ ID N° : 2 ; qui est fonctionnellement lié à un acide nucléique hétérologue codant pour une protéine immunogène.

16. La composition immunothérapeutique de la revendication 15, qui est un vaccin.

17. Composition immunothérapeutique comprenant une bactérie transformée avec une construction d'expression, comprenant un promoteur d'origine bactérienne, qui est régulable par des conditions d'inanition, et comprend : une séquence de nucléotides illustrée dans la SEQ ID N° : 1 ou la SEQ ID N° : 2 ; au moins un fragment actif de promoteur correspondant à la SEQ ID N° : 1 ou la SEQ ID N° : 2 ; ou une séquence de nucléotides au moins 60 % identique à la SEQ ID N° : 1 ou la SEQ ID N° : 2 ; qui est fonctionnellement lié à un acide nucléique hétérologue codant pour une protéine immunogène, ensemble avec un véhicule, un diluant ou un excipient pharmaceutiquement acceptable.

18. La composition immunothérapeutique de la revendication 17, dans laquelle ladite bactérie est atténuée.

19. La composition immunothérapeutique de la revendication 17, dans laquelle ladite bactérie est d'une souche de *Salmonella sp.*

20. La composition immunothérapeutique de la revendication 17, dans laquelle ladite construction d'expression est intégrée et conservée dans un chromosome de ladite bactérie.

21. La composition immunothérapeutique de la revendication 17, dans laquelle ladite construction d'expression est conservée à l'extérieur d'un chromosome dans ladite bactérie.

22. La composition immunothérapeutique de la revendication 17, qui est un vaccin.

23. L'utilisation d'une construction d'expression, comportant un promoteur d'origine bactérienne, régulable par des conditions d'inanition, et comprenant : une séquence de nucléotides illustrée dans la SEQ ID N° : 1 ou la SEQ ID N° : 2 ; au moins un fragment actif de promoteur correspondant à la SEQ ID N° : 1 ou la SEQ ID N° : 2 ; ou une séquence de nucléotides au moins 60 % identique à la SEQ ID N° : 1 ou la SEQ ID N° : 2 ; fonctionnellement lié à un acide nucléique hétérologue qui code pour une protéine immunogène appropriée à la thérapie et/ou la prophylaxie d'un ou plusieurs états morbides sensibles à une immunothérapie chez un hôte, pour la préparation d'un médicament destiné au traitement et/ou la prophylaxie d'un ou plusieurs états morbides.

24. L'utilisation d'une construction d'expression contenant un promoteur d'origine bactérienne régulable par des conditions d'inanition, et comprenant : une séquence de nucléotides illustrée dans la SEQ ID N° : 1 ou la SEQ ID N° : 2 ; au moins un fragment actif de promoteur correspondant à la SEQ ID N° : 1 ou la SEQ ID N° : 2 ; ou une séquence de nucléotides au moins 60 % identique à la SEQ ID N° : 1 ou la SEQ ID N° : 2 ; fonctionnellement lié à un acide nucléique hétérologue qui code pour une protéine immunogène, pour la préparation d'un médicament destiné à la vaccination d'un hôte.

25. L'utilisation de la revendication 24, dans laquelle une réponse immune protectrice est provoquée chez ledit hôte.

26. L'utilisation de l'une quelconque des revendications 23 à 25, dans laquelle la construction d'expression est présente dans une bactérie atténuée.

27. Utilisation de la revendication 26, dans laquelle la bactérie est une souche de *Salmonella sp.*

28. L'utilisation selon l'une quelconque des revendications 23 à 25, dans laquelle ledit hôte est un humain.
